# EUROPEAN PATENT SPECIFICATION

(11) **EP 1 165 095 B1**
(45) Date of publication and mention of the grant of the patent: **28.05.2008**
(21) Application number: 00916034.2
(22) Date of filing: 02.03.2000
(51) Int. Cl.: A61K 31/70, A61K 31/60, A61K 31/495, A61K 31/40, A61K 31/195, A61K 31/19, A61K 31/17

(54) **AGENTS FOR INTRAVITREAL ADMINISTRATION TO TREAT OR PREVENT DISORDERS OF THE EYE**
MITTEL FÜR INTRAVITREALE VERABREICHUNG ZUR BEHANDLUNG ODER VORBEUGUNG VON AUGENERKRANKUNGEN
AGENTS DESTINES A ETRE ADMINISTRES PAR VOIE INTRAVITREENNE POUR TRAITER OU PREVENIR DES TROUBLES OCULAIRES

(30) Priority: 02.03.1999 US 122503 P
(43) Date of publication of application: 02.01.2002
(73) Proprietor: Vitreo-Retinal Technologies, Inc., Chula Vista, CA 91910 (US)
(72) Inventor: CASTILLEJOS, David, Chula Vista, CA 91910 (US)
(74) Representative: Whitaker, Iain Mark
(86) International application number: PCT/US2000/005587
(87) International publication number: WO 2000/051620

(56) References cited:
- EP-A- 0 244 178
- US-A- 5 110 493
- US-A- 5 441 984
- US-A- 5 554 187
- US-A- 5 891 084
- US-A- 5 891 913
- DATABASE BIOSIS [Online] BIOSCIENCES INFORMATION SERVICE, PHILADELPHIA, PA, US; 1989, SHIMADA H ET AL: "EFFECTS OF AN ANTI-PROSTAGLANDIN AGENT ADDED TO THE IRRIGATION SOLUTION ON DAMAGE TO THE ANTERIOR SEGMENT IN MONKEY EYES INDUCED BY PARS PLANA VITRECTOMY" XP002301766 Database accession no. PREV199089052309 & NIPPON GANKA GAKKAI ZASSHI, vol. 93, no. 8, 1989, pages 823-829, ISSN: 0029-0203
- DATABASE BIOSIS [Online] BIOSCIENCES INFORMATION SERVICE, PHILADELPHIA, PA, US; 1996, KAWASHIMA SHIGENOBU ET AL: "Effects of mitomycin C on the rat retina" XP002301767 Database accession no. PREV199799620113 & DOCUMENTA OPHTHALMOLOGICA, vol. 92, no. 3, 1996, pages 229-241, ISSN: 0012-4486
- ALLEN J B ET AL: "Nitric oxide synthase inhibitors exert differential time-dependent effects on LPS-induced uveitis." EXPERIMENTAL EYE RESEARCH. JAN 1996, vol. 62, no. 1, January 1996 (1996-01), pages 21-28, XP002301765 ISSN: 0014-4835

## Description

### FIELD OF THE INVENTION

The present invention relates generally to pharmaceutical preparations and medical treatment methods, and more particularly agents (i.e, urea and urea derivatives, nonsteroidal anti-inflammatory drugs (NSAIDS) and anti -metabolite drugs) used alone or in combinations with each other (or with other agents) to treat or prevent certain disorders of the eye.

### BACKGROUND OF THE INVENTION

### A. Neovascularization

Neovascularization (or angiogenesis) is a process whereby new blood vessels are formed within tissues of the body. *Normal* neovascularization is the physiological process by which the body creates and maintains small blood vessels of the circulatory system. However, *pathological* or *iatrogenic* neovascularization is a non-physiological process whereby abnormal networks of blood vessels are created in tissues of the body or in tumors, as a result of certain diseases, trauma or surgical procedures.

Pathological neovascularization occur within tissues of the eye as a result of certain ophthalmic disorders such as diabetic retinopathies, proliferative vitreo-retinopathies, corneal neovascularization, iris rubeosis, and diseases that cause ischemia of the ocular tissues (e.g., occlusion of the central retinal vein, occlusion of the central retinal artery, certain inflammatory conditions, etc..). Also, iatrogenic neovascularization can occur following certain ophthalmological surgical procedures which disrupt normal blood supply to tissues of the eye or those which cause localized proliferations of cells known as "fibroblasts". Examples of ophthalmological surgical procedured that have been associated with untoward post-surgical neovascularization include glaucoma filtration surgery and corneal transplant surgery.

Various types of drugs and agents (e.g., steroids, non-steroidal anti-inflammatory drugs (NSAIDS), heparin, protamine, calcitrol, antibiotics, thrombospodin fragments, and monoclonal antibodies directed to fibroblast growth factor) have been purported to be useable to treat or prevent neovascularization of the anterior segment of the eye. Non steriodal anti-inflammatory drugs have not been used to treat neovascularization of the posterior segment of the eye.

Also, laser surgical procedures have been used to ablate or destroy neo-vascular networks which develop in the retina or epiretinal membranes. However, these prior therapies for ocular neovascularization have been less than completely effective and/or have been associated with side effects. For example, glucocorticoids and other angiostatic steroids have been used to treat neovascularization of the anterior chamber (e.g., corneal neovascularization, iris rubeosis) and/or other ocular tissues, but such steroid treatments have been associated with side effects such as elevated intraoccular pressure. *see*, Kitazawa, Increased Intraocular Pressure Induced by Corticosteroids, American Journal of Ophthalmology, Vol. 82, Pg.492-493 (1976)

### B. Intravitreal Hemorrhage and the Need for Liquefaction of the Vitreous Body and/or Posterior Vitreous Dissinsertion or Detachment Prior to Vitrectomy:

In many mammals including human beings, the "vitreous body" is disposed within a posterior portion of the eye and occupies approximately four fifths of the cavity of the eyeball, behind the lens. The vitreous body is formed of gelatinous material, known as the vitreous humor. The vitreous humor of a normal human eye is made up of approximately 99% water along with 1% macromolecules including; collagen, hyaluronic acid, soluble glycoproteins, sugars and other low molecular weight metabolites.

The retina is essentially a layer of nervous tissue which covers a portion of the inner wall of the posterior segment-in juxtaposition to the posterior aspect of the vitreous body. The retina is surrounded by a layer of cells known as the choroid layer. The retina may be divided into a) an optic portion which participates in the visual mechanism, and b) a non-optic portion which does not participate in the visual mechanism. The optic portion of the retina contains the rods and cones, which are the effectual organs of vision. A number of arteries and veins enter the retina at its center, and splay outwardly to provide blood circulation to the retina.

The posterior portion of the vitreous body is in direct contact with the retina. Networks of fibrillar strands extend from the retina and permeate or insert into the vitreous body so as to attach the vitreous body to the retina.

Diabetic retinopathy, trauma and other ophthalmological disorders sometimes result in rupture or leakage of retinal blood vessels with resultant bleeding into the vitreous humor of the eye (i.e., "intravitreal hemorrhage). Such intravitreal hemorrhage typically manifests as clouding or opacification of the vitreous humor.

Intravitreal hemorrhage is sometimes, but not always, accompanied by tearing or detachment of the retina. In cases where the intravitreal hemorrhage is accompanied by a retinal tear or detachment, it is important that such retinal tear or detachment be promptly diagnosed and surgically repaired. Failure to promptly diagnose and repair the retinal tear or detachment may allow photo-receptor cells of the retina, in the region of the tear or detachment, to become necrotic. Such necrosis of the photoreceptor cells of the retina may result in loss of vision. Furthermore, allowing the retinal detachment to remain unrepaired for such extended period of time may result in further intravitreal hemorrhage and/or the formation of fibrous tissue at the site of the hemorrhage. Such formation of fibrous tissue may result in the formation of an undesirable fibrous attachment between the vitreous body and the retina.

The typical surgical procedure used for repair of retinal tears or detachment requires that the surgeon be able to look through the vitreous humor, to visualize the damaged region of the retina (i.e., "transvitreous viewing of the retina"). When intravitreal hemorrhage has occurred, the presence of the hemorrhagic blood within the vitreous can cause the vitreous to become so cloudy that the surgeon is prevented from visualizing the retina through the vitreous. Such hemorrhagic clouding of the vitreous can take 6-12 months or longer to clear sufficiently to permit trans-vitreal viewing of the retina. However, in view of the potential complications which may result from delayed diagnosis or treatment of a retinal tear or detachment, it is generally not desirable to wait for such natural clearance of the hemorrhagic blood to occur.

Furthermore, even when the intravitreal hemorrhage is not accompanied by retinal tear or detachment, it is often difficult to verify that retinal tear or detachment has not occurred, because the hemorrhagic clouding of the vitreous prevents the physician from performing routine funduscopic examination of the retina. Moreover, the presence of hemorrhagic blood within the vitreous may significantly impair the patient's vision through the affected eye, and will continue to do so until such time as the hemorrhagic blood has been substantially or fully cleared.

Thus, the presence of hemorrhagic blood within the vitreous body causes multiple clinical problems including a) inability to visually examine and diagnose the site of the hemorrhage and/or any accompanying tear or detachment of the retina, b) full or partial impairment of vision in the affected eye and c) impairment or prevention of the performance of trans-vitreal surgical procedures of the type typically utilized to repair the site of hemorrhage and/or to repair any accompanying retinal tear or detachment. In cases where intravitreal hemorrhage has resulted in substantial clouding or opacification of the vitreous, the treating physician may have the option to perform a procedure known as a vitrectomy, wherein all (or a portion of) the vitreous body is removed from the interior of the eye, and replaced with a clear liquid or gas. The performance of such vitrectomy procedure is intended to allow the surgeon to visualize the retina sufficiently to proceed with the necessary retinal examination and/or surgical repair of the hemorrhage and any accompanying retinal tear or detachment. However, such vitrectomy procedures are highly skill-intensive, and are associated with several significant drawbacks, risks and complications. Among these drawbacks, risks and complications are the potential that the act of removing the vitreous will cause further detachment or tearing of the retina and/or that such removal of the vitreous will cause further hemorrhage from the already-weakened retinal blood vessels. In order to minimize the stress of tugging on the retina during vitrectomy and to otherwise facilitate the removal of the vitreous body, it is sometimes desirable to precede the vitrectomy by the intravitreal injection of a substance which will cause liquefaction of the vitreous humor and/or disinsertion/detachment of the vitreous humor from the adjacent tissues of the retina and epiretinal membranes. Examples of substances which have been purported to cause vitreal liquefaction and/or posterior vitreous detachment/disinsertion are found in United States Patent Nos. 4, 820, 516 (Sawyer), 5,292,509 (Hageman) and 5, 866, 120 (Karageozian et al.).

### C. Prior Ophthalmic Applications of Urea and Urea Derivatives:

United States Patents No. 5,629,344 (Charlton et al.) has described the topical application to the cornea or "surface" of the eye of urea and/or urea derivative(s) to treat ocular conditions such as dryness, non-infectious keratitis, irregularities of the corneal or conjunctival epithelium, ocular scarring and "subjective irritations" as well as to inhibit unwanted fibroblast formation and/or enhance healing following glaucoma, cataract and corneal surgeries.

### D. Prior Ophthalmic Applications of Non-Steroidal Anti-Inflammatory Agents:

Several NSAIDS have heretofore been known for oral administration and/or topical application to the eye for the purpose of treating inflammatory conditions of the eye and/or post surgical pain and inflamation of the anterior segment of the eye. Examples of NSAIDS that are presently available for topical application to the eye include diclofenac (Cataflam), flurbiprofen (Ansaid), ketorolac (Toradol, Acular). To date, ophthalmic NSAID preparations have been used primarily to treat inflammatory disorders of the cornea and anterior segment of the eye. In instances where ophthalmic NSAIDS have been administered to treat disorders of the posterior segment of the eye, the NSAID has been initially applied to the anterior segment of the eye with the intention that a therapeutic amount of the NSAID will distribute from the anterior segment to the posterior segment of the eye where the therapeutic effect is desired. For example, Acular^{™} (ketorolac) drops have been applied topically to the anterior segment of the eye (i.e., to the cornea) for the purpose of treating cystoid macular edema--a disorder of the posterior segment of the eye.

### E. Prior Ophthalmic Applications of Anti-Metabolites:

5-Fluorouracil and Mitomicin C have previously been purported to be useable, when administered to the anterior segment of the eye, to inhibit or treat certain conditions of the anterior segment that are characterized by undesired tissue proliferation such as benign fibrovascular lesions of the conjunctiva known as Pterygia.

### SUMMARY OF THE INVENTION

According to the present invention there is provided the use of urea or a urea derivative as defined in Claim 1.

Also described is a method of treatment which generally comprises the step of delivering into the eye (e.g., by intravitreal injection or instillation into the posterior segment of the eye) a therapeutically effective amount of an agent selected from the following:
a) urea
b) urea derivatives (e.g., hydroxyurea, thiourea);
c) a non-steroidal anti-inflammatory agent;
d) an antmetabolite;
e) a urea, urea derivative, non-enzymatic protein urea, urea derivatives, non-enzymatic proteins, nucleosides, nucleotides and their derivatives (e.g., adenine, adenosine, cytosine, cytadine, guanine, guanitadine, guanidinium, thymidine, thimitadine, uradine, uracil, cystine), uric acid, calcium acetal salicylate, ammonium sulfate or other compound capable of causing non-enzymatic dissolution of the hyaloid membrane; and,
f) any of the possible combinations thereof.

The method is carried out for the purpose of bringing about one or more of the following effects:
- causing non-exzymatic dissolution of the hyaloid interface (i.e., hyaloid membrane) or other proteins or amino acids responsible for maintaining attachment between the vitreous body and retina of a mammalian eye, or otherwise inducing posterior vitreous detachment and/or disinsertion of the vitreous body from the retina and epiretinal membranes (hereinafter referred to as "PVD");
- causing liquefaction of the vitreous humor;
- causing diffusion or preventing the accumulation of localized concentrations near the retina of substances that are injurious or pathogenic to the retina (e.g., angiogenic factors);
- causing dissolution of coagulum within the vitreous humor (as may occur following intravitreal hemorrhage);
- causing a solvent action on fibroblasts;
- inhibiting fibroblasts;
- inhibiting or preventing fibrosis associated with the presence of vitreous heme;
- inhibiting the proliferation of fibroblasts in ocular tissues; and,
- causing reactivation (e.g., regeneration, regrowth, stimulation, up-regulation or improved neuronal transmission) of inactive nerves or nerve fibers (e.g., optic nerve).

By one or more of the above-listed effects, or by other mechanisms, the method of is useable for various therapeutic and/or prophylactic applications, including but not limited to:
- treating (e.g., as used herein "treating" shall mean preventing, deterring, stopping, curing or slowing the progression of) diabetic retinopathy;
- treating intravitreal hemorrhage and accelerating the clearance of hemorrhagic blood from the vitreous burner;
- inducing PVD and/or liquefaction of the vitreous prior to the performance of a vitrectomy thereby limiting the likelihood of retinal detachment, retinal tearing, re-stimulation of retinal hemorrhage or other complications of the vitrectomy procedure;
- treating vitreous traction associated with macular holes;
- treating macular degeneration;
- treat retinitis pigmentosa;
- prophylaxis to retinal detachment in patients who are at high risk for retinal detachment (e.g, high myopes);
- treating preretinal and subretinal membranes;
- treating cystoid macular edema;
- pre-operative preparation of the eye in the surgical treatment of eye trauma;
- pre-operative treatment prior to certain types of glaucoma surgery (e.g., those performed for the treatment of neovascular glaucoma);
- treating occlusion of the central retinal vein or central retinal artery;
- treating conditions associated with neovascularization such as neovascular iris and neovascular glaucoma;
- treating ocular ischemic syndrome;
- treating conditions associated with posterior eye inflamation such as VKH, pars planitis, toxoplasmosis, etc.; and,
- improving the delivery and bioavilability to the retina and other tissues of intravitreally administered drugs;
- treating pterygia (e.g., loxopterygium, pimelopterygium, symblepharopterygium);
- treating stromal-comeal neovascularization;
- treating glaucoma blebs;
- treating optic nerve atrophy or impaired optic nerve activity of any cause; and,
- treating glaucoma-induced cupping of the optic nerve.

Further in accordance with the invention, the urea and urea derivative agents useable in the method comprise urea, hydroxyurea and thiourea.

Still further in accordance with the invention, the agents of the present invention may be administered by injection into the eye (e.g., intravitreal, intrastromal or sub-conjunctival injection).

Still further in accordance with the invention, the non-steroidal anti-inflammatory drugs that are most suitable for use in the method of the present invention include the heteroaryl acetic acids (e.g., tolmetin, diclofenac, ketorolac) and the arylpropionic acids (e.g., ibuprofen, naproxen, flurbiprofen, ketoprofen, fenoprofen, oxaprozin). Combination of an NSAID with urea or a urea-containing compound is particularly useable for the treatment of posterior inflammatory conditions such as VKH, pars planitis, toxoplasmosis, etc.

Still further in accordance with the invention, the anti-metabolite compounds that are useable comprise, mitomicyn C, methotrexate, thiourea, hydroxyurea, 6-mercaptopurine, thioguanine, 5-fluorouracil, cytosine arabinoside and 5-azacytidine. (Thiourea and hydroxyurea are anti-metabolites *as well* as urea derivatives.) Other anti-neoplastic compound such as Actinomycin D, daunorubicin, doxorubicin, idarubicin, bleomycins, or plicamycin may also be used in combination with these anti-metabolites. Combinations of these anti-metabolite agents (or other anti-neoplastic drugs) with urea or a urea-containing compounds are particularly useable for the treatment of Intraocular tumors, posterior uveitis, wet macular degeneration, age related macular degeneration (dry form), retinitis pigmentosa, retina of prematurity (ROP), retinal vasculitis (e.g., secondary to Eales disease, lupus retinopathy, sarcoidosis, etc.), neovascular glaucoma, phacomorphic reactions and sympathetic ophthalmia.

Still further in accordance with the invention, PVD may be induced by the administration (e.g. intravitreal injection) of therapeutically effective amount(s) of one or more agents that cause non-enzymatic dissolution of the hyaloid membrane or hyaloid interface. As a result of such hyaloid dissolution, the vitreous body will become detached or disinserted from the retina, thereby allowing vitrectomy, repair of retinal tears, or other procedure to be performed with lessened chance for inducing retinal tearing or retinal hemorrhage. For example, in many traditional vitrectomy procedures, the tugging or cutting away of the vitreous body can result in tearing or damage to the retina because the hyaloid interface remains in tact. Also, after the vitreous body has been substantially removed using a vitrectomy cutter, the physician must (in many cases) peal the remaining hyaloid membrane away from the retina. Such pealing away of the hyaloid from the retina can further cause retinal tearing or damage. Thus, by administering one or more compounds capable of causing non-enzymatic dissolution of the hyaloid membrane, PVD may be induced and such potential for iatrogenic damage to the retina will be minimized. Examples of agents that may be administered by intravitreal injection to cause such non-enzymatic dissolution of the hyaloid membrane include urea, urea derivatives, urea, urea derivatives, non-enzymatic proteins, nucleosides, nucleotides and their derivatives (e.g., adenine, adenosine, cytosine, cytadine, guanine, guanitadine, guanidinium, thymidine, thimitadine, uradine, uracil, cystine), uric acid, calcium acetal salicylate and ammonium sulfate.

Still further aspects, objects and advantages of the invention will be apparent to those of skill in the art who read and understand the following detailed description of the invention and the specific examples set forth therein.

### DETAILED DESCRIPTION OF THE INVENTION

As summarized hereabove, the present invention provides urea containing solutions (i.e., solutions which contain urea, a urea derivative (e.g., hydroxyurea) and/or mixtures thereof) that are injectable into the eye alone, non-steroidal anti-inflammatory drugs (NSAIDS) injected into the eye alone and anti-metaboilities that are injected into the eye alone. Additionally, the some of the urea-containing or injectable solutions of the present invention may further contain non-steroidal anti-inflammatory agent(s) (e.g., flurbiprofen, diclofenac, ketorolac) and/or antimetabolite(s) (e.g., mitomicyn C, methotrexate, 6-mercaptopurine, thioguanine, 5-fluorouracil, cytosine arabinoside and 5-azacytidine).

Solutions of urea or hydroxyurea, which have been adjusted to a pH of approximately 4.0 to 7.0, are substantially non-toxic and well tolerated when injected intravitrially, sub-conjunctively or intrastromally, one (1), two (2) or more times, in an injectate volume of 50-100 microliters per injection, at doses of 33-5000 micrograms of urea per injection.

### A. Urea Formulations

The following are examples of urea-containing solutions that are useable in accordance with this invention:

### Example 1

| | |
|---|---|
| Urea USP/NF | 4.0% |
| Sodium Chloride USP/NF | up to 0.9% |
| Sterile Water for Injection USP | Q.S. 100% |
| pH of solution | 5.0- 7.0 |
| (Adjust pH using 0. 1 N Hydrochloric acid 0. 1 N Sodium Hydroxide) | |

### Example 2

| | |
|---|---|
| Urea USP/NF | 4.0% |
| Citric Acid USP/NF | 0.00007-0.02% |
| Sterile Water for Injection USP | Q.S. 100% |
| pH of solution | 5.0- 7.0 |
| (Adjust pH using 0. 1 N Hydrochloric acid 0. 1 N Sodium Hydroxide) | |

### Example 3

| | |
|---|---|
| Urea USP/NF | 0.01% - 15.0% |
| Sodium Chloride USP/NF | up to 0.9% |
| Sterile Water for Injection USP | Q.S. 100% |
| pH of solution | 5.0- 7.0 |
| (Adjust pH using 0. 1 N Hydrochloric acid / 0. 1 N Sodium Hydroxide) | |

### Example 4

| | |
|---|---|
| Urea USP/NF | 4.0% |
| Potassium Phosphate Dibasic USP/NF | 5.0 millimolar |
| Sterile Water for Injection USP | Q.S. 100% |
| pH of solution | 5.0- 7.0 |
| (Adjust pH using 0. 1 N Hydrochloric acid/ 0. 1 N Sodium Hydroxide) | |

### Example 5

| | |
|---|---|
| Urea USP/NF | 4.0% |
| Potassium Phosphate Dibasic USP/NF | 50.0 millimolar |
| Sterile Water for Injection USP | Q.S. 100% |
| pH of solution | 5.0- 7.0 |
| (Adjust pH using 0. 1 N Hydrochloric acid / 0. 1 N Sodium Hydroxide) | |

### Example 6 (Lyophilized Powder)

| | |
|---|---|
| Urea USP/NF | 0.01%-15.0% |
| Sorbitol USP/NF | 0.10%-0.50% |
| Citric Acid USP/NF | 0.00007-0.02% |
| pH of final solution | 5.0- 7.0 |
| (Adjust pH using 0. 1 N Hydrochloric acid / 0. 1 N Sodium Hydroxide) | |

### Example 7

| | |
|---|---|
| Urea USP/NF | 4.0% |
| Sorbitol USP/NF | 0.10% |
| Citric Acid USP/NF | 0.00007-0.02% |
| pH of final solution | 5.0- 7.0 |
| (Adjust pH using 0. 1 N Hydrochloric acid / 0. 1 N Sodium Hydroxide) | |

Citrate or phosphate buffers may alternatively be used in the above-listed formulations of Examples 1-7. Also, sodium chloride and dextrose are alternative bulking agents that could be used in the lyophilized formulation of Example 6.

### B. Urea-Enzyme Solutions

The following are examples of formulations wherein urea is combined with another agent, such as an enzyme.

### Example 8

| | |
|---|---|
| Urea USP/NF | 4.0 % |
| Hyaluronidase | 1.0 IU-100 IU |
| Sodium Chloride USP/NF | up to 0.9 % |
| Sterile Water for Injection USP | Q.S. 100% |
| pH of solution | 5.0- 7.0 |
| (Adjust pH using 0. 1 N Hydrochloric acid 0. 1 N Sodium Hydroxide) | |

### Example 9

| | |
|---|---|
| Urea USP/NF | 0.01 % -15.0 % |
| Hyaluronidase | 1.0 IU- 100 IU |
| Sodium Chloride USP/NF | up to 0.9 % |
| Sterile Water for Injection USP | Q.S. 100% |
| pH of solution | 5.0- 7.0 |
| (Adjust pH using 0. 1 N Hydrochloric acid/ 0. 1 N Sodium Hydroxide) | |

### Example 10

| | |
|---|---|
| Urea USP/NF | 0.01 % -15.0 % |
| Urokinase | 1.0 IU- 50 IU |
| Sodium Chloride USP/NF | up to 0.9 % |
| Sterile Water for Injection USP | Q.S. 100% |
| pH of solution | 5.0- 7.0 |
| (Adjust pH using 0. 1 N Hydrochloric acid/ 0. 1 N Sodium Hydroxide) | |

### Example 11

| | |
|---|---|
| Urea USP/NF | 0.01 % -15.0 % |
| Chondroitinase ABC | 1.0 IU- 30 IU |
| Sodium Chloride USP/NF | up to 0.9 % |
| Sterile Water for Injection USP | Q.S. 100% |
| pH of solution | 5.0- 7.0 |
| (Adjust pH using 0. 1 N Hydrochloric acid 0. 1 N Sodium Hydroxide) | |

### Example 12

| | |
|---|---|
| Hydroxy Urea | 0.01 % - 15.0 % |
| Hyaluronidase. | 1.0 IU- 100 IU |
| Sodium Chloride USP/NF | up to 0.9 % |
| Sterile Water for Injection USP | Q.S. 100% |
| pH of solution | 5.0- 7.0 |
| (Adjust pH using 0. 1 N Hydrochloric acid/ 0. 1 N Sodium Hydroxide) | |

### C. Hydroxy Urea Solutions

The following are examples of hydroxy urea-containing formulations useable in accordance with the present invention.

### Example 13

| | |
|---|---|
| Hydroxy Urea | 4.0% |
| Sodium Chloride USP/NF | up to 0.9% |
| Sterile Water for Injection USP | Q.S. 100% |
| pH of solution | 5.0- 7.0 |
| (Adjust pH using 0. 1 N Hydrochloric acid / 0. 1 N Sodium Hydroxide) | |

### Example 14

| | |
|---|---|
| Hydroxy Urea | 4.0% |
| Citric Acid USP/NF | 0.00007-0.02% |
| Sterile Water for Injection USP | Q.S. 100% |
| pH of solution | 5.0- 7.0 |
| (Adjust pH using 0. 1 N Hydrochloric acid / 0. 1 N Sodium Hydroxide) | |

### Example 15

| | |
|---|---|
| Hydroxy Urea | 0.01% - 15.0% |
| Sodium Chloride USP/NF | up to 0.9% |
| Sterile Water for Injection USP | Q.S. 100% |
| pH of solution | 5.0- 7.0 |
| (Adjust pH using 0. 1 N Hydrochloric acid / 0. 1 N Sodium Hydroxide) | |

### Example 16

| | |
|---|---|
| Hydroxy Urea | 4.0% |
| Potassium Phosphate Dibasic USP/NF | 5.0 millimolar |
| Sterile Water for Injection USP | Q.S. 100% |
| pH of solution | 5.0- 7.0 |
| (Adjust pH using 0. 1 N Hydrochloric acid 0.1 N Sodium Hydroxide) | |

### Example 17

| | |
|---|---|
| Hydroxy Urea | 4.0% |
| Potassium Phosphate Dibasic USP/NF | 50.0 millimolar |
| Sterile Water for Injection USP | Q.S. 100% |
| pH of solution | 5.0- 7.0 |
| (Adjust pH using 0. 1 N Hydrochloric acid 0. 1 N Sodium Hydroxide) | |

### Example 18 (Lyophilized Powder)

| | |
|---|---|
| Hydroxy Urea | 0.01% - 15.0% |
| Sorbitol USP/NF | 0.10% - 0.50% |
| Citric Acid USP/NF | 0.00007-0.02% |
| pH of final solution | 5.0- 7.0 |
| (Adjust pH using 0. 1 N Hydrochloric acid 0. 1 N Sodium Hydroxide) | |

### Example 19

| | |
|---|---|
| Hydroxy Urea | 4.0% |
| Sorbitol USP/NF | 0.10% |
| Citric Acid USP/NF | 0.00007-0.02% |
| pH of final solution | 5.0- 7.0 |
| (Adjust pH using 0. 1 N Hydrochloric acid 0. 1 N Sodium Hydroxide) | |

### D. Urea-NSAID and Hydroxy Urea-NSAID Solutions

The following are examples of formulations for Urea-NSAID and Hydroxy Urea-NSAID Solutions that are useable in accordance with the present invention.

### Example 20 (not encompassed by the scope of the claims)

| | |
|---|---|
| Flurbiprofen Sodium | 0.03% |
| Sodium Chloride USP/NF | 0.70% |
| Citrate buffer | 5.0 - 50.0 millimolar |
| Sterile Water for Injection USP | Q.S. 100% |
| pH of solution | 4.0 - 6.0 |
| (Adjust pH using 0. 1 N Hydrochloric acid 0. 1 N Sodium Hydroxide) | |

### Example 21

| | |
|---|---|
| Flurbiprofen Sodium | 0.03% |
| Urea USPNF | 4.0% |
| Citrate buffer | 5.0 - 50.0 millimolar |
| Sterile Water for Injection USP | Q.S. 100% |
| pH of solution | 4.0 - 6.0 |
| (Adjust pH using 0. 1 N Hydrochloric acid / 0. 1 N Sodium Hydroxide) | |

### Example 22

| | |
|---|---|
| Flurbiprofen Sodium | 0.03% |
| Hydroxy Urea | 4.0% |
| Citrate buffer | 5.0 - 50.0 millimolar |
| Sterile Water for Injection USP | Q.S. 100% |
| pH of solution | 4.0 - 6.0 |
| (Adjust pH using 0. 1 N Hydrochloric acid / 0. 1 N Sodium Hydroxide) | |

### Example 23

| | |
|---|---|
| Flurbiprofen Sodium | 0.01% - 0.03% |
| Urea USP/NF | 0.01% - 15.0% |
| Citrate buffer | 5.0 - 50.0 millimolar |
| Sterile Water for Injection USP | Q.S. 100% |
| pH of solution | 4.0 - 6.0 |
| (Adjust pH using 0. 1 N Hydrochloric acid / 0. 1 N Sodium Hydroxide) | |

### Example 24

| | |
|---|---|
| Flurbiprofen Sodium | 0.01% - 0.03% |
| Hydroxy Urea | 0.01% - 15.0% |
| Citrate buffer | 5.0 - 50.0 millimolar |
| Sterile Water for Injection USP | Q.S. 100% |
| pH of solution | 4.0 - 6.0 |
| (Adjust pH using 0. 1 N Hydrochloric acid / 0. 1 N Sodium Hydroxide) | |

It will be appreciated that any of the above-set-forth solutions may be injected intravitreously or may be injected into other portions of the eye, to effect the therapeutic treatment(s) of the present invention.

### E. Antibetabolite Formulations:

The following are examples of formulations for antimetabolite solutions that are useable in accordance with the present invention.

### Example 25

| | |
|---|---|
| Hydroxyurea | 0.01% - 15.0% |
| NaCl USP/NF | up to 0.9% |
| Sterile Water for Injection USP | Q.S. 100% |
| pH | 5.0-7.0 |
| (Adjust pH using 0.1 N Hydrochloric acid 0.1 N Sodium Hydroxide) | |

### Example 26 (not encompassed by the scope of the claims)

| | |
|---|---|
| Mitomycin C | 200 µg - 200 mg |
| NaCl USP/NF | up to 0.9% |
| Sterile Water for Injection | Q.S. 100% |
| pH | 5.5-7.5 |
| (Adjust pH using 0.1 N Hydrochloric acid 0.1 N Sodium Hydroxide) | |
| (Refrigerate after compounding, stable for up to 3 days) | |

### Example 27 (not encompassed by the scope of the claims)

| | |
|---|---|
| Mitomycin C | 200 µg - 200 mg |
| Sorbital USP/ NF | 0.1 - 0.5% |
| NaCl (lyophilized powder) | 0.2% |

### Example 28

| | |
|---|---|
| Thiourea | 0.01% - 10.0% |
| NaCl USP/NF | up to 0.9% |
| Sterile Water for Injection | Q.S. 100% |
| pH | 4.0 - 6.0 |
| (Adjust pH using 0.1 N Hydrochloric acid 0.1 N Sodium Hydroxide) | |

### G. Other Agents for Non-Enzymatic Dissolution of Hyaloid Interface:

As summarized hereabove, any of the above formulations containing urea or a urea derivative may be administered by intravitreal injection or otherwise caused to distribute into the vitreous or posterior segment of the eye in therapeutic concentrations, to cause non-enzymatic dissolution of the hyaloid membrane, thereby inducing PVD. In combination with, urea or a urea derivative, these formulations may contain other compounds capable of causing non-enzymatic dissolution of the hyaloid membrane such as non-enzymatic proteins, nucleosides, nucleotides and their derivatives (e.g., adenine, adenosine, cytosine, cytadine, guanine, guanitadine, guanidinium, guanidinium, thymidine, thimitadine, uradine, uracil, cystine), uric acid, calcium acetal salicylate and ammonium sulfate. For example, a guanidinium preparation of the following formulation may be administered by intravitreal injection, to deliver a dose of approximately 30micrograms to 5 milligrams, and preferably about 2mg. The injectate volume in which each such intravitreal dose is delivered is preferably about 50miroliters per injection. As a result of one or more intravitreal injections of this guanidinium preparation, the hyaline membrane will be substantially dissolved resulting in substantial PVD.

### Example 33

| | |
|---|---|
| Guanidinium HCL | 0.01 %-15.0% |
| Sodium Chloride USP/NF | up to 0.9% |
| Sterile Water for Injection USP | O.S. 100% |
| pH of solution | 5.0- 7.0 |
| (Adjust pH using 0. 1 N Hydrochloric acid 0. 1 N Sodium Hydroxide) | |

It is to be appreciated that the invention has been described hereabove with reference to certain presently preferred embodiments and examples only, and no effort has been made to exhaustively describe all possible embodiments and examples wherein urea containing solutions (e.g. urea or hydroxy urea solutions) and/or solutions containing compounds capable of inducing non-enzymatic dissolution of the hyaloid membrane are used in accordance with the present invention. It is intended that all possible embodiments and examples of such solutions and the above-stated uses thereof, be included within the scope of following claims:

## Claims

1. The use of a therapeutically effective amount of urea, a urea derivative or a combination of urea and a urea derivative, in the preparation of a medicament for delivery into the vitreous humor or posterior segment of the eye to treat or prevent disorders including: to treat or prevent proliferation of fibroblasts within the eye; to treat or prevent neovascularisation of ocular tissues; to accelerate the clearance of haemorrhagic blood from the vitreous humor; to cause non-enzymatic dissolution of the hyaloid interface; and/or to cause posterior vitreous detachment or disinsertion.

2. The use according to claim 1, wherein said medicament is in an injectable form for delivery into the posterior segment of the eye by injection.

3. The use according to claim 2, wherein said medicament is in a form for intravitreal injection.

4. The use according to any preceding claim 1 wherein the medicament contains a urea derivative selected from the group consisting of:
hydroxyurea;
thiourea; and
possible combinations thereof.

5. The use according to any preceding claim wherein the medicament contains the urea, urea derivative or combination of urea and a urea derivative in an amount that is effective to cause non-enzymatic dissolution of the hyaloid membrane.

6. The use according to claim 3 wherein the medicament comprises 30 micrograms -7500 micrograms of urea per 50 microliters to 100 UL. of solution.

7. The use according to claim 5 wherein the medicament comprises 300 micrograms of urea per 50 microliters of solution.

8. The use according to claim 3 wherein the medicament delivers a dose of 0.01% to 15.0% urea into the vitreous body of the eye.

9. The use according to claim 1 wherein the medicament comprises at least one additional agent selected from the group consisting of; non-steroid anti-inflammatory agents; antimetaboiites; nucleosides; nucleotides; adenine; adenosine; cytosine; cytadine; guanine; guanitadine; guanidinium; thymidine; thimitadine; uradine; uracil; cystine; uric acid; calcium acetal salicylate; ammonium sulfate; and combinations thereof.

10. The use according to claim 9 wherein the medicament comprises i) urea, a urea derivative or a combination of urea and a urea derivative, in combination with ii) a non-steroidal anti-inflammatory agent selected from the group consisting of:
flurbuprofen;
diclofenac; and
ketorolac

11. The use according to claim 9 wherein the medicament comprises i) urea, a urea derivative or a combination of urea and a urea derivative, in combination with ii) at least one antimetabolite.

12. The use according to claim 11 wherein the antimetabolite is selected from the group consisting of:
mitomicyn C;
methotrexate;
6-mecaptopurine;
thioguanine;
5-fluorouracil;
cytosine arabinoside;
5-azacytidine;
hydroxyurea;
thiourea; and
possible combinations thereof.

13. The use according to claim 11 or claim 12 wherein the medicament comprises 2000 micrograms of urea and either 2000 micrograms of antimetabolite hydroxyurea or 5.0 microgram of mitomicyn C per 50 microliters of solution.

14. The use according to claim 11 or claim 12 wherein the medicament comprises 300 micrograms of urea and 2000 micrograms of hydroxyurea, or 10 micrograms mitomicyn C per 50 microliters of solution.

15. The use according to any of claims 11 to 14 inclusive wherein the medicament is administered a plurality of times to deliver a dose of 2000 micrograms urea and 5.0 micrograms of an anti-metabolite per delivery.

16. The use according to claim 1 wherein the medicament may be administered to treat or prevent said disorder by causing an effect selected from the group consisting of:
inducing posterior vitreous detachment and/or disinsertion of the vitreous body from the retina and epiretinal membranes (hereinafter referred to as "PVD");
causing non-enzymatic dissolution of the hyaloid membrane; causing liquefaction of the vitreous humor;
causing diffusion of preventing the accumulation of localised concentrations near the retina of substances that are injurious or pathogenic to the retina (e.g. angiogenic factors);
causing dissolution of coagulum within the vitreous humor (as may occur following intravitreal hemorrhage);
causing a solvent action on fibroblasts; inhibiting fibroblasts;
inhibiting or preventing fibrosis associated with the presence of vitreous heme;
inhibiting the proliferation of fibroblasts in ocular tissues;
treating diabetic retinopathy;
accelerating the clearance of hemorrhagic blood from the vitreous humor;
inducing PVD and/or liquefaction of the vitreous prior to the performance of a vitrectomy thereby limiting the likelihood of retinal detachment, retinal tearing, restimulation of retinal hemorrhage or other complications of the vitrectomy procedure;
treating vitreous traction associated with macular hole;
treating macular degeneration;
treating retinitis pigmentosa;
providing prophylaxis to retinal detachment in patients who are at high risk for retinal detachment;
treating preretinal and subretinal membranes;
treating cystoid macular edema;
pre-operative preparation of the eye in the surgical treatment of eye trauma;
pre-operative treatment prior to certain types of glaucoma surgery (e.g. those performed for the treatment of neovascular glaucoma);
treating occlusion of the central retinal vein or central retinal artery;
treating conditions associated with posterior eye inflammation such VKH, pars pianitis, toxoplasmosis, etc. and
improving the delivery and bioavailability to the retina and other tissues of intravitreally administered drugs.

17. The use according to any preceding claim wherein said medicament is delivered into the posterior segment of the eye by initially administering the agent to the anterior segment of the eye in a form and dose that is sufficient to cause a therapeutic amount of the agent to distribute to the posterior segment.

## Patentansprüche

1. Verwendung einer therapeutisch wirksamen Menge von Harnstoff, eines Harnstoff-Derivats oder einer Kombination von Harnstoff und eines Harnstoff-Derivats bei der Zubereitung eines Medikaments zur Verabreichung in dem Glaskörper oder das hintere Segment des Auges zur Behandlung oder zur Verhinderung von Erkrankungen, unter Einschluss von: der Behandlung oder Verhinderung der Ausbreitung von Fibroblasten innerhalb des Auges; zur Behandlung oder Verhinderung von neuem Gefäßwachstum von Augengeweben; zur Beschleunigung der Entfernung von hämorrhagischem Blut aus dem Glaskörper; zum Bewirken einer nicht-enzymatischen Auflösung der Hyaloid-Grenzfläche; und/oder zum Hervorrufen einer posteriosen Glaskörper-Ablösung oder Abtrennung.

2. Verwendung nach Anspruch 1, bei der das Medikament in einer injizierbaren Form zur Verabreichung in das posteriore Segment des Auges durch eine Injektion vorliegt.

3. Verwendung nach Anspruch 2, bei der das Medikament von einer intravitrealen Injektion vorliegt.

4. Verwendung nach einem der vorhergehenden Ansprüche, bei der das Medikament ein Harnstoff-Derivat enthält, dass aus der Gruppe ausgewählt ist, die aus Folgendem besteht:
Hydroxyurea;
Thiouria; und
möglichen Kombinationen hiervon.

5. Verwendung nach einem der vorhergehen Ansprüche, bei der das Medikament den Harnstoff, das Harnstoff-Derivat oder die Kombination von Harnstoff und einem Harnstoff-Derivat in einer Menge enthält, die zum Hervorrufen einer nicht-enzymatischen Auflösung der Hyaloid-Membran wirksam ist.

6. Verwendung nach Anspruch 3, bei dem das Medikament 30 Mikrogramm bis 7500 Mikrogramm an Harnstoff pro 50 Mikroliter bis 100 Mikroliter der Lösung enthält.

7. Verwendung nach Anspruch 5, bei der das Medikament 300 Mikrogramm von Harnstoff pro 50 Mikroliter der Lösung enthält.

8. Verwendung nach Anspruch 3, bei der das Medikament eine Dosis von 0,01 % bis 15,0°/a Harnstoff in den Glaskörper des Auges verabreicht.

9. Verwendung nach Anspruch 1, bei dem das Medikament zumindest ein zusätzliches Mittel umfasst, das aus der Gruppe ausgewählt ist, die aus Folgenden besteht: nicht-steroide entzündungshemmende Mittel; Antimetabolit; Nucleosid, Nucleotid, Adenin, Adenosin; Zytosin; Zytadin, Guanin, Guanitadin, Guanidin, Thymidin, Thymitadin; Uradin; Urazil; Zystin; Harnsäure; Kalziumazetalsalizylat; Ammoniumsulfat; und Kombinationen hiervon.

10. Verwendung nach Anspruch 9, bei dem das Medikament i) Harnstoff, ein Harnstoff-Derivat oder eine Kombination von Harnstoff und einem Harnstoff-Derivat in Kombination mit ii) einen nicht-steroiden entzündungshemmenden Mittel umfasst, das aus der Gruppe ausgewählt ist, die aus Folgendem besteht:
Flurbiprofen;
Diclofenac; und
Ketorolac.

11. Verwendung nach Anspruch 9, bei dem das Medikament i) Harnstoff, ein Harnstoff-Derivat oder eine Kombination von Harnstoff und einem Harnstoff-Derivat in Kombination mit ii) mindestens einen Antimetaboliten umfasst.

12. Verwendung nach Anspruch 11 bei dem der Antimetabolit aus der Gruppe ausgewählt ist, die aus Folgendem besteht:
Mitomycin C;
Methotrexat;
6-Mercaptopurine;
Thioguanin;
5-Fluorouracil;
Cytosinarabinosid;
5-Azacytidin;
Hydroxyharnstoff;
Thioharnstoff; und
mögliche Kombinationen hiervon.

13. Verwendung nach Anspruch 11 oder Anspruch 12, bei dem das Medikament 2000 Mikrogramm von Harnstoff und entweder 2000 Mikrogramm von Antimetabolit, Hydroxyharnstoff oder 5,0 Mikrogramm von Mitomicyn C pro 50 Mikroliter der Lösung umfasst.

14. Verwendung nach Anspruch 11 oder Anspruch 12, bei dem das Medikament 300 Mikrogramm an Harnstoff und 2000 Mikrogramm an Hydroxyharnstoff oder 10 Mikrogramm Mitomicyn C pro 50 Mikroliter an Lösung umfasst.

15. Verwendung nach einem der Ansprüche 11 bis 14 einschließlich, bei der das Medikament mehrere Male verabreicht wird, um eine Dosis von 2000 Mikrogramm Harnstoff und 5,0 Mikrogramm eines Antimetaboliten pro Verabreichung zuzuführen.

16. Verwendung nach Anspruch 1, bei dem das Medikament verabreicht werden kann, um die Erkrankung zu behandeln oder zu verhindern, indem ein Effekt hervorgerufen wird, der aus der Gruppe ausgewählt ist, die aus Folgendem besteht:
Hervorrufen einer posterioren Glaskörper-Abtrennung und/oder Ablösung des Glaskörpers von der Hornhaut und dem epiretinalen Membran (nachfolgend als "PVD" bezeichnet);
Hervorrufen einer nicht-enzymatischen Auflösung der Hyaloid-Membran; Hervorrufen einer Verflüssigung des Glaskörpers;
Hervorrufen einer Diffusion oder Verhindern der Akkumulation von lokalisierten Konzentrationen in der Nähe der Hornhaut von Substanzen, die für die Hornhaut schädlich oder pathogen sind (beispielsweise angiogene Faktoren);
Hervorrufen einer Auflösung eines Koagulums innerhalb des Glaskörpers (wie er nach einer intravitrealen Hemorrhage auftreten kann);
Hervorrufen einer Lösewirkung auf Fibroblasten; Verhindern von Fibroblasten;
Unterdrücken oder Verhindern einer Fibrose, die mit dem Vorhandensein eines Glaskörper-Häms verbunden ist;
Unterdrücken der Verbreitung von Fibroblasten in Augengewebe;
Behandeln einer diabetischen Retinopathie;
Beschleunigen des Entfernens von hemorrhagischen Blut aus dem Glaskörper;
Hervorrufen von PVD und/oder einer Verflüssigung des Glaskörpers vor der Durchführung einer Vitrektomie, wodurch die Wahrscheinlichkeit einer Hornhaut-Abtrennung, Hornhaut-Ablösung, Neu-Stimulation einer retinalen Hemorrhagie oder andere Komplikationen des Vitrektomie-Verfahren begrenzt wird;
Behandeln der Glaskörper-Spannung, die mit der Makular-Öffnung verbunden ist;
behandeln der Makulardegeneration;
behandeln der Retinitis Pigmentosa;
Bereitstellung einer Prophylaxe für die Hornhaut-Ablösung bei Patienten, die ein hohes Risiko der Hornhaut-Ablösung haben;
Behandlung der präretinalen und subretinalen Membranen;
Behandlung von zystoiden Makular-Ödemen;
vor-operative Vorbereitung des Auges bei einer chirurgischen Behandlung von Augen-Traumata;
vor-operative Behandlung vor bestimmten Arten der Glaukom-Chirurgie (beispielsweise diejenigen, die für die Behandlung von neovaskularen Glaukomen ausgeführt werden);
Behandlung des Verschlusses der zentralen retinalen Vene oder der zentralen retinalen Arterie;
Behandlung von Bedingungen, die mit einer posterioren Augenentzündung verbunden sind, wie zum Beispiel VKH, Pars Pianitis, Toxoplasmose, usw.; und
Verbessern der Verabreichung und Bioverfügbarkeit für die Hornhaut und andere Gewebe von intravitreal verabreichten Medikamenten

17. Verwendung nach einem der vorhergehenden Ansprüche, bei der das Medikament in das posteriore Segment des Auges durch anfängliches Verabreichen des Mittels an das vordere Segment des Auges in einer Form und Dosis verabreicht wird, die ausreicht, damit sich eine therapeutische Menge des Mittels zu dem posterioren Segment verteilt.

## Revendications

1. Utilisation d'une quantité efficace d'un point de vue thérapeutique d'urée, d'un dérivé d'urée ou d'une combinaison d'urée et d'un dérivé d'urée, dans la préparation d'un médicament destiné à être administré dans l'humeur vitrée ou un segment postérieur de l'oeil pour traiter ou empêcher des troubles y compris, traiter ou empêcher une prolifération de fibroblastes à l'intérieur de l'oeil ; traiter ou empêcher une néovascularisation de tissus oculaires ; accélérer l'épuration de sang hémorragique de l'humeur vitrée ; provoquer une dissolution non-enzymatique de l'interface hyaloïde ; et/ou provoquer un décollement vitréen postérieur ou une désinsertion.

2. Utilisation selon la revendication 1, dans laquelle ledit médicament est sous une forme injectable destinée à être administrée dans le segment postérieur de l'oeil par injection.

3. Utilisation selon la revendication 2, dans laquelle ledit médicament est sous une forme destinée à une injection intravitréenne.

4. Utilisation selon l'une quelconque des revendications précédentes, dans laquelle le médicament contient un dérivé d'urée sélectionné dans le groupe comprenant :
de l'hydroxyurée ;
de la thiourée ; et
leurs combinaisons possibles.

5. Utilisation selon l'une quelconque des revendications précédentes, dans laquelle le médicament contient de l'urée, un dérivé d'urée ou une combinaison d'urée et d'un dérivé d'urée en une quantité qui est efficace pour provoquer une dissolution non-enzymatique de la membrane hyaloïde.

6. Utilisation selon la revendication 3, dans laquelle le médicament comprend 30 microgrammes à 7 500 microgrammes d'urée par 50 microlitres à 100 µL de solution.

7. Utilisation selon la revendication 5, dans laquelle le médicament comprend 300 microgrammes d'urée par 50 microlitres de solution.

8. Utilisation selon la revendication 3, dans laquelle le médicament administre une dose de 0,01 % à 15,0 % d'urée dans le corps vitré de l'oeil.

9. Utilisation selon la revendication 1, dans laquelle le médicament comprend au moins un agent additionnel sélectionné dans le groupe comprenant : des agents anti-inflammatoires non-stéroïdiens; des antimétabolites ; des nucléosides ; des nucléotides ; de l'adénine; de l'adénosine; de la cytosine; de la cytadine; de la guanine ; de la guanitadine ; du guanidinium ; de la thymidine ; de la thimitadine ; de l'uradine ; de l'uracile ; de la cystine ; de l'acide urique ; de l'acétalsalicylate de calcium ; du sulfate d'ammonium ; et leurs combinaisons.

10. Utilisation selon la revendication 9, dans laquelle le médicament comprend i) de l'urée, un dérivé d'urée ou une combinaison d'urée et d'un dérivé d'urée, en combinaison avec ii) un agent anti-inflammatoire non-stéroïdien sélectionné dans le groupe comprenant :
flurbiprofène ;
diclofénac ; et
kétorolac

11. Utilisation selon la revendication 9, dans laquelle le médicament comprend i) de l'urée, un dérivé d'urée ou une combinaison d'urée et d'un dérivé d'urée, en combinaison avec ii) au moins un antimétabolite.

12. utilisation selon la revendication 11, dans laquelle l'antimétabolite est sélectionné dans le groupe comprenant :
mitomycine C ;
méthotrexate ;
6-mercaptopurine ;
thioguanine ;
5-fluoro-uracile ;
cytosine-arabinoside ;
5-azacytidine ;
hydroxyurée ;
thiourée ; et
leurs combinaisons possibles.

13. Utilisation selon la revendication 11 ou 12 dans laquelle le médicament comprend 2 000 microgrammes d'urée et soit 2 000 microgrammes d'hydroxyurée antimétabolite soit 5,0 microgrammes de mitomycine C par 50 microlitres de solution.

14. Utilisation selon la revendication 11 ou 12, dans laquelle le médicament comprend 300 microgrammes d'urée et 2 000 microgrammes d'hydroxyurée, ou 10 microgrammes de mitomycine C par 50 microlitres de solution.

15. Utilisation selon l'une quelconque des revendications 11 à 14 incluses, dans laquelle le médicament est délivré une pluralité de fois pour administrer une dose de 2 000 microgrammes d'urée et de 5,0 microgrammes d'un antimétabolite par administration.

16. Utilisation selon la revendication 1, dans laquelle le médicament peut être délivré pour traiter ou empêcher ledit trouble en provoquant un effet sélectionné dans le groupe consistant en les étapes suivantes :
induire un décollement vitréen postérieur et/ou une désinsertion du corps vitré de la rétine et des membranes épirétinales (dénommées ci-après PVD) ;
provoquer une dissolution non-enzymatique de la membrane hyaloïde ; provoquer une liquéfaction de l'humeur vitrée ;
provoquer une diffusion consistant à empêcher l'accumulation de concentrations localisées près de la rétine de substances qui sont nuisibles ou pathogènes pour la rétine (par exemple, des facteurs angiogéniques) ;
provoquer une dissolution d'un caillot à l'intérieur de l'humeur vitrée (tel qu'il peut en apparaître après une hémorragie intravitréenne) ;
provoquer une action de dilution sur des fibroblastes ;
inhiber des fibroblastes ; inhiber ou empêcher une fibrose associée à la présence d'un hème vitré ;
inhiber la prolifération de fibroblastes dans des tissus oculaires ;
traiter une rétinopathie diabétique;
accélérer l'épuration de sang hémorragique de l'humeur vitrée ;
induire un PVD et/ou une liquéfaction du corps vitré avant la réalisation d'une vitrectomie, limitant de ce fait la probabilité de décollement rétinien, de déchirement rétinien, de restimulation d'une hémorragie rétinienne ou d'autres complications de la procédure de vitrectomie ;
traiter une traction vitreuse associée à un trou maculaire ;
traiter une dégénérescence maculaire ;
traiter une rétinite pigmentaire ;
fournir une prophylaxie pour un décollement rétinien chez des patients présentant un risque élevé de décollement rétinien ;
traiter des membranes pré-rétiniennes et subrétiniennes ;
traiter l'oedème masculaire cystoïde ;
préparation pré-opératoire de l'oeil dans le traitement chirurgique d'un traumatisme oculaire ;
traitement pré-opératoire avant certains types de chirurgie du glaucome (par exemple, celles effectuées pour le traitement d'un glaucome néovasculaire) ;
traiter une occlusion de la veine centrale de la rétine ou de l'artère centrale de la rétine ;
traiter des troubles associés à une inflammation oculaire postérieure telle que le syndrome de VKH, la pars planitis, la toxoplasmose, etc. et
améliorer l'administration et la biodisponibilité pour la rétine et d'autres tissus de médicaments délivrés par voie intravitréenne.

17. Utilisation selon l'une quelconque des revendications précédentes, dans laquelle ledit médicament est administré dans le segment postérieur de l'oeil en délivrant initialement l'agent au segment antérieur de l'oeil sous une forme et une dose qui suffisent pour provoquer la distribution d'une quantité thérapeutique de l'agent au segment postérieur.
